# EUROPEAN PATENT SPECIFICATION

(11) **EP 3 554 408 B1**
(45) Date of publication and mention of the grant of the patent: **22.04.2020**
(21) Application number: 17821838.4
(22) Date of filing: 08.12.2017
(51) Int. Cl.: A61B 18/20, A61B 18/00

(54) **A CUTTING ELEMENT FOR A HAIR CUTTING DEVICE**
SCHNEIDEELEMENT FÜR EINE HAARSCHNEIDEVORRICHTUNG
ÉLÉMENT DE COUPE POUR UN DISPOSITIF DE COUPE DE CHEVEUX

(30) Priority: 13.12.2016 EP 16203753
(43) Date of publication of application: 23.10.2019
(73) Proprietor: Koninklijke Philips N.V., 5656 AG Eindhoven (NL)
(72) Inventor: VERHAGEN, Rieko, 5656 AE Eindhoven (NL); MOESKOPS, Bastiaan Wilhelmus Maria, 5656 AE Eindhoven (NL); THUMMA, Kiran Kumar, 5656 AE Eindhoven (NL); JOHNSON, Mark Thomas, 5656 AE Eindhoven (NL); BOAMFA, Marius Iosif, 5656 AE Eindhoven (NL)
(74) Representative: Uittenbroek, Arie Leendert
(86) International application number: PCT/EP2017/081947
(87) International publication number: WO 2018/108719

(56) References cited:
- WO-A1-2014/143670
- US-A1- 2008 244 912
- US-A1- 2013 050 831

## Description

### FIELD OF THE INVENTION

The invention relates to a cutting assembly for use with a hair cutting device for cutting (e.g. shaving) hair on a body of a subject and, in particular, a cutting assembly having structures formed therein. The invention also relates to a hair cutting device comprising such a cutting assembly.

### BACKGROUND OF THE INVENTION

A shaving device has been proposed in WO 2014/143670 that makes use of laser light. In particular, a laser light source is provided that is configured to generate laser light having a wavelength selected to target a predetermined chromophore to effectively cut a hair shaft. A fibre optic is located on a shaving portion of the device that is positioned to receive the laser light from the laser light source at a proximal end, conduct the laser light from the proximal end toward a distal end, and emit the light out of a cutting region of the fibre optic and toward hair when the cutting region is brought in contact with the hair.

Laser light propagates through multimode optical fibre in a large number of propagation modes. Light propagating in some of these propagation modes is more likely to couple out from the optical fibre into hair than light in other propagation modes.

### SUMMARY OF THE INVENTION

In order to cut or melt hair, sufficient optical energy needs to couple out from a surface of a cutting element of the cutting device into hair to initiate the cutting/melting of hair. Laser light propagating through the optical waveguide of the cutting element in relatively lower-order propagation modes is likely to propagate nearer to the centre of the optical waveguide and substantially parallel to an optical axis of the optical waveguide and, therefore, is less likely to couple out from a surface of the optical waveguide into a hair.

Thus, there exists a need for a cutting element from which a greater amount of light is able to couple out into hair to be cut. In other words, there exists a need for a more efficient cutting element.

According to a first aspect, there is provided a cutting element for use in a hair cutting device, the cutting element comprising an optical waveguide having an optical axis and a sidewall, wherein a portion of the sidewall forms a cutting face for contacting hair; wherein the optical waveguide includes a plurality of structures formed along the optical axis, the structures having a refractive index which is different to a refractive index of the optical waveguide.

By forming structures having a lower refractive index along the optical axis, some of the light propagating through the optical waveguide will interact with the structures, causing it to reflect, refract and/or scatter so that it propagates at a greater angle with respect to the optical axis. This causes more light to be available at the cutting face for coupling out into hairs, thereby improving the cutting effectiveness of the cutting element.

Each structure may comprise one of a cavity, a void or a bubble. Each structure may be substantially spherical. Cavities formed within the optical waveguide are not affected by factors such as erosion caused by use in a wet environment and, therefore, forming such structures should have little detrimental effect, if any, on the lifetime of the cutting element.

In some embodiments, the refractive index of the structures may be lower than the refractive index of the optical waveguide. By forming the structures such that they have a refractive index which is lower than the refractive index of the optical waveguide, the desired effect on the direction of the light is achieved. Specifically, the angle with respect to the optical axis at which some of the light propagates through the optical waveguide is increased.

The structures may be regularly spaced along the optical axis of the optical waveguide. In some embodiments, the structures may be substantially the same size as one another.

The optical waveguide may, in some embodiments, comprise an optical fibre. The optical waveguide may be formed from a material selected from a group comprising: quartz, sapphire, yttrium aluminium garnet, YAG, yttrium aluminosilicates, alumino silicates, alkaline earth silicates, alkali aluminosilicates, rare-earth doped aluminosilicates, and oxyfluorides.

Each structure may have a size which is smaller than a size of a lowest-order light propagation mode of the optical waveguide.

According to a second aspect, there is provided a hair cutting device for cutting hair on a body of a subject, the hair cutting device comprising a light source for generating laser light at one or more specific wavelengths corresponding to wavelengths absorbed by one or more chromophores in hair; and a cutting element as described herein, the cutting element being coupled to the light source.

According to a third aspect, there is provided a method of manufacturing an optical waveguide cutting element for use in a hair cutting device, the method comprising providing an optical waveguide having an optical axis; determining a pattern in which to form a plurality of structures within the optical waveguide; focusing a laser beam within the optical waveguide so as to form the plurality of structures along the optical axis in accordance with the determined pattern.

The determining may be based at least in part on at least one of: a length of the cutting element, a diameter of the cutting element, a material from which the cutting element is formed, and a wavelength of light to be propagated through the optical waveguide.

Other advantageous features will become apparent from the following description.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a block diagram of a hair cutting device according to embodiments of the invention;
Fig. 2 is a pair of schematic drawings showing different views of an exemplary hair cutting device according to embodiments of the invention;
Fig. 3 is a graph illustrating the refractive index of hair;
Fig. 4 is a schematic illustration of an example of a portion of a cutting element according to embodiments of the invention;
Fig. 5 is a schematic illustration of a further example of a portion of a cutting element according to embodiments of the invention; and
Fig. 6 is a flowchart showing an example of a method of manufacturing an optical waveguide cutting element for use in a hair cutting device.

### DETAILED DESCRIPTION OF THE EMBODIMENTS

As noted above, the present invention provides an improvement in the cutting ability and efficiency of a laser light-based shaving device, for example as described in WO 2014/143670. In particular, it has been recognised that cutting efficiency can be improved by introducing a plurality of structures along the optical axis of an optical waveguide cutting element, to increase the amount of light reaching the sidewall of the optical waveguide. By increasing the amount of light able to couple out from the cutting element into hair, it may be possible to cut more hair in the same, or in a shorter amount of time, resulting in a more rapid and efficient hair cutting experience. Consequently, the need for a user to repeatedly use the shaving device over the same area of his or her skin is reduced, along with the risk of pain or irritation of the skin.

It will be appreciated that the invention is applicable to shaving devices (e.g. razors or electric shavers), and any other type of device that is used to cut hair (e.g. hair clippers), even if those devices do not necessary aim to provide a 'clean shave' (i.e. to remove hair at the level of the skin).

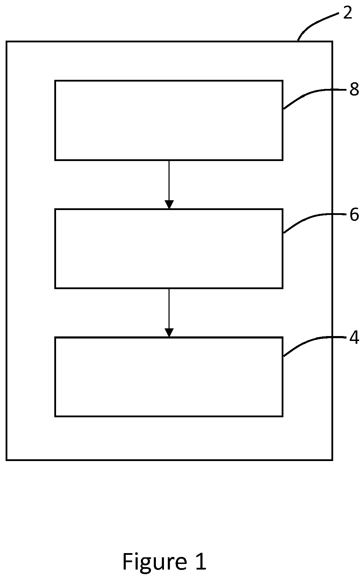

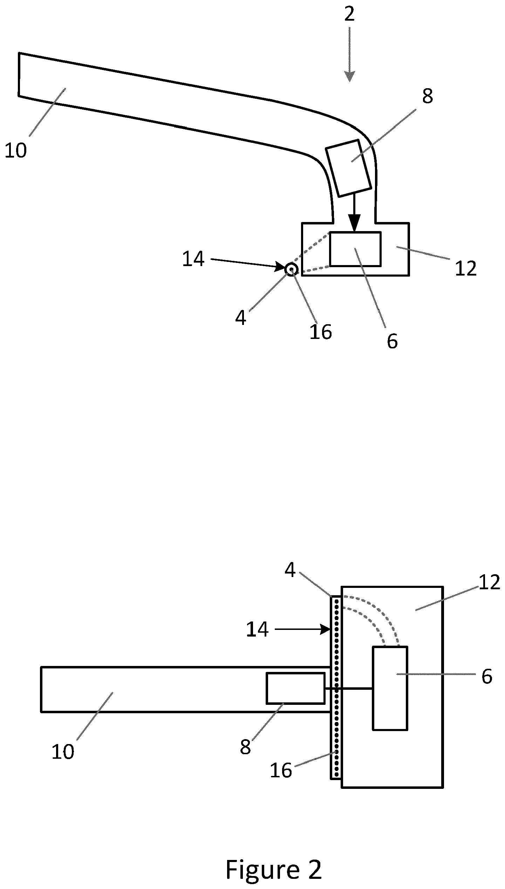

Fig. 1 is a block diagram of a hair cutting device 2 according to an embodiment of the invention. Fig. 2 shows a hair cutting device 2 in the form of a handheld razor according to an exemplary embodiment of the invention. The hair cutting device 2 is for cutting (e.g. shaving) hair on a body of a subject. The subject may be a person or an animal. The hair may be facial hair (i.e. hair on the subject's face), or hair on the subject's head or other part of their body (legs, chest, etc.).

The hair cutting device 2 comprises a cutting element 4 that enables hair to be cut as the hair cutting device 2 is moved over the skin of a subject. The cutting element 4 is an optical waveguide 4 that is arranged on the hair cutting device 2 so that the optical axis of the optical waveguide 4 (i.e. the line along which light typically propagates through the optical waveguide 4) is generally perpendicular to the direction in which the hair cutting device 2 is moved so that hairs contact the sidewall of the optical waveguide 4 (the sidewall corresponding to the long edge of the optical waveguide 4) as the hair cutting device 2 is moved across the skin of the subject. In some embodiments, the optical waveguide 4 is an optical fibre, although those skilled in the art will be aware of other types of optical waveguide that can be used according to the invention, such as a slab waveguide, a strip waveguide or a photonic crystal waveguide. An optical fibre comprises a core, and in some embodiments also comprises a cladding, which may or may not fully encompass the core (e.g. part of the core may be exposed).

A light source 6 is provided in the hair cutting device 2 that generates laser light at one or more specific wavelengths. The light source 6 is optically coupled to the optical waveguide 4 so that the laser light generated by the light source 6 is coupled into the optical waveguide 4 (and specifically coupled into at least one end of the optical waveguide 4 so that the laser light propagates through the optical waveguide 4).

The light source 6 is configured to generate laser light at one or more specific wavelengths that can be used to cut or burn through hair. In particular, each wavelength corresponds to the wavelength of light absorbed by a chromophore that is found in hair. As is known, a chromophore is the part of a molecule that provides the molecule with its colour. Thus, the laser light will be absorbed by the chromophore and converted into heat which will melt or burn the hair or otherwise destroy the bonds in the molecules of the hair, and it is this melting or burning that provides the cutting action of the hair cutting device 2.

Suitable chromophores that can be targeted by the laser light generated by the light source 6 include, but are not limited to, melanin, keratin and water. Suitable wavelengths of laser light that can be used include, but are not limited to, wavelengths selected from the range 380 nm (nanometres) to 500 nm and 2500 nm to 3500 nm. Those skilled in the art will be aware of the wavelengths of light that are absorbed by these chromophores, and thus also the specific wavelengths of light that the light source 6 should generate for this purpose, and further details are not provided herein.

In some embodiments the light source 6 can be configured to generate laser light at a plurality of wavelengths (either simultaneously or sequentially), with each wavelength being selected to target a different type of chromophore. This can improve the cutting action of the optical waveguide 4 since multiple types of molecules in the hair may be burnt using the laser light. Alternatively multiple light sources 6 can be provided that each generate laser light at a respective wavelength. The multiple light sources may be coupled to a single optical waveguide, or each light source 6 can be coupled to a respective optical waveguide 4 to provide multiple cutting elements 4 in the device 2.

The hair cutting device 2 also comprises a control unit 8 that controls the operation of the hair cutting device 2, and in particular is connected to the light source 6 to control the activation and deactivation of the light source 6 (and in some embodiments control the wavelength and/or intensity of the light generated by the light source 6). The control unit 8 may activate and deactivate the light source 6 in response to an input from a user of the hair cutting device 2. The control unit 8 can comprise one or more processors, processing units, multi-core processors or modules that are configured or programmed to control the hair cutting device 2.

As noted above, Fig. 2 shows a hair cutting device 2 that is in the form of a handheld wet razor. Fig. 2 shows a side view and a bottom view of the razor 2. The razor 2 comprises a handle 10 for the subject (or other user of the device 2) to hold, and a head portion 12 that includes the cutting element 4 (optical waveguide/fibre). As shown, the optical waveguide 4 is arranged along an edge of the head portion, and a part of the optical waveguide 4 forms (or corresponds to) a cutting face 14. The cutting face 14 is the part of the optical waveguide 4 that is intended to come into contact with hair as the hair cutting device 2 is moved across the skin of the subject. A plurality of structures 16 are formed along the optical axis of the optical waveguide 4, the purpose of which is discussed below. A light source 6 and control unit 8 are shown as being incorporated into the head portion 12 and handle 10 respectively, but it will be appreciated that the positions of these components in the hair cutting device 2 as shown in Fig. 2 is not limiting. Likewise it will be appreciated that the embodiment shown in Fig. 2 is merely an example, and the invention can be incorporated or used in any type of hair cutting device 2 that comprises an optical waveguide cutting element 4 as described herein.

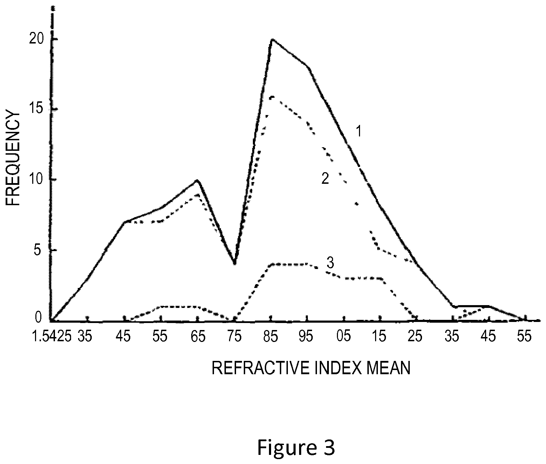

The graph in Fig. 3 illustrates the refractive index of hair, which can be found in a paper by M. D. Greenwell, A. Willner, Paul L. Kirk: Human Hair Studies: III. Refractive Index of Crown Hair, 31 Am. Inst. Crim. L. & Criminology 746 (1940-1941). Curve 1 is a composite line, curve 2 is a line representing the refractive index for Caucasian people, and curve 3 is a line representing the refractive index for non-Caucasian people. Thus, it can be seen that the refractive index of hair is between (approximately) 1.545 and 1.555, although there will be variation between individuals. For example the above paper also recognises that the refractive index of hair can depend on the sex of the subject, e.g. the refractive index of hair on a female is generally higher than the refractive index of hair on a male.

As is known, the optical waveguide 4 acts as a waveguide for the light coupled from the light source 6 through the occurrence of total internal reflection, since the refractive index of air is lower than that of the optical waveguide 4. However, if an object that has a refractive index higher than the optical waveguide 4 is put into contact with the optical waveguide 4, then the total internal reflection is 'frustrated' and light can couple from the optical waveguide 4 into that object. Thus, in order for light to be coupled into a hair from the optical waveguide 4 (to provide the cutting action according to the invention), the optical waveguide 4 should preferably have the same or a lower refractive index than hair at the point at which the hair contacts the optical waveguide 4. Thus, the optical waveguide 4 should preferably have the same or a lower refractive index than hair at least at the cutting face 14 portion of the optical waveguide 4. Preferably the refractive index of the optical waveguide 4 at the cutting face 14 is the same as that of hair since that provides the best coupling of light from the optical waveguide 4 to the hair. Light may still be able to couple from the optical waveguide 4 into an object (e.g. a hair) brought into contact with the cutting face 14 of the optical waveguide even if the refractive index of the optical waveguide is higher than that of the object, due to a high numerical aperture in the cutting face.

Thus, in some embodiments, the refractive index of the optical waveguide 4 at least at the cutting face 14 is equal to or lower than 1.56. More preferably the refractive index of the optical waveguide 4 at least at the cutting face 14 is equal to or lower than 1.55. Even more preferably, the refractive index of the optical waveguide 14 at least at the cutting face 14 is equal to or lower than 1.54, since this refractive index is below the refractive indices identified in Fig. 3.

In some embodiments, a lower bound for the refractive index of the optical waveguide 4 at the cutting face 14 can be 1.48, 1.51, 1.53 or 1.54.

A range of values from which the refractive index of the optical waveguide 4 is selected can be formed from any combination of the upper and lower refractive index bounds set out in the preceding paragraphs.

The optical waveguide/fibre 4 can be made from any suitable material or combination of materials. For example optical waveguides/fibres can be composed of or comprise silica, fluoride glass, phosphate glass, chalcogenide glass, crown glass (such as BK7), quartz, and/or crystals (such as yttrium aluminium garnet (YAG), sapphire, yttrium aluminosilicates, aluminosilicates, alkaline earth silicates, alkali aluminosilicates, rare-earth doped aluminosilicates, and oxyfluorides).

In some embodiments, the optical waveguide 4 of the cutting element may comprise an optical fibre having an yttrium aluminium garnet (YAG) core with a quartz cladding. YAG has a relatively high refractive index. In scenarios where laser light having a wavelength of 445 nanometres is propagated through a YAG optical waveguide, the refractive index is around 1.85. With laser light having a wavelength of 1 micrometre (µm), the refractive index of YAG is around 1.82, and with laser light having a wavelength of 2 µm, the refractive index is around 1.80. In scenarios where laser light having a wavelength of 445 nanometres is propagated through a quartz optical waveguide, the refractive index is around 1.47. With laser light having a wavelength of 1 micrometre (µm), the refractive index of quartz is around 1.45, and with laser light having a wavelength of 2 µm, the refractive index is around 1.44. Laser light coupled into the optical waveguide 4 from the laser source 6 will propagate through the core of the optical waveguide in a number of propagation modes, each propagation mode corresponding to a particular propagation angle relative to the optical axis (or to a normal of the optical axis) of the optical waveguide. As is known, light may propagate through the optical waveguide at angles (relative to the optical axis of the waveguide) up to a numerical aperture (NA) of the optical waveguide, which is the maximum range of angles at which light is able to couple into the waveguide. Light propagating in higher-order propagation modes propagates at a relatively large angle with respect to the optical axis. Conversely, light propagating in lower-order propagation modes propagates at a relatively small angle with respect to the optical axis. Furthermore, light propagating in lower-order propagation modes tends to propagate through the central region of the optical waveguide 4, so there is less interaction with light in the lower-order modes with the surface of the optical waveguide and, therefore, less opportunity for light in the lower-order modes to couple out into hairs.

The inventors have discovered that, by forming a plurality of structures along the optical axis of the optical waveguide 4 (e.g. along the centre of an optical fibre), the structures having a refractive index which is different to a refractive index of the optical waveguide, some of the light propagating along the optical waveguide (for example, the light propagating in the lower-order propagation modes) may be deflected, refracted, scattered or bent such that the propagation angle relative to the optical axis is increased. In other words, the numerical aperture of light encountering the structures is increased.

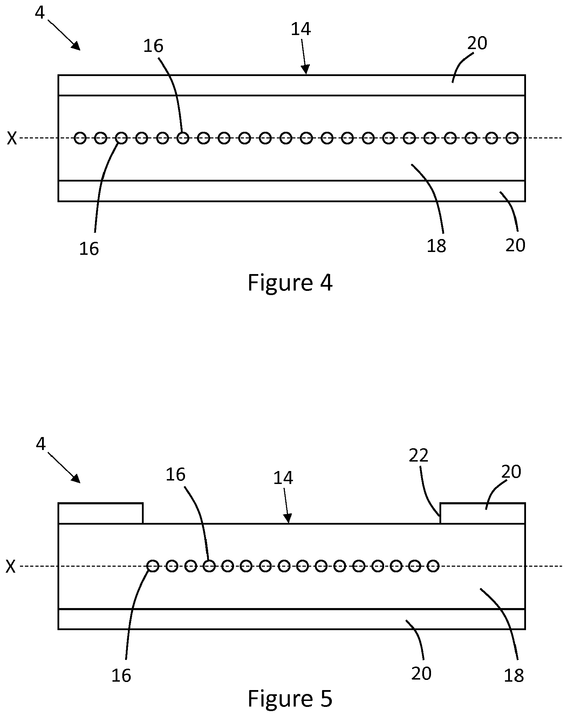

Fig. 4 shows, schematically, a cross section of a portion of a cutting element 4 for use in a hair cutting device, according to the invention. The cutting element 4 comprises an optical waveguide having an optical axis X and a sidewall, wherein a portion of the sidewall forms a cutting face 14 for contacting hair. In this embodiment, the structures comprise a plurality of cavities 16 formed along the optical axis of the optical waveguide 4. The optical waveguide 4 shown in Fig. 4 comprises a core 18 and a cladding 20 surrounding the core. Thus, in this embodiment, the cutting face 14 comprises a portion of an outer surface of the cladding 20.

An alternative embodiment is shown in Fig. 5, in which a portion of the cladding 20 of the optical waveguide 4 has been removed in a region 22 of the waveguide, to expose the core 18. In this embodiment, a surface of the core 18 forms the cutting face 14 of the cutting element. Removing a portion of the cladding 20 of the optical waveguide can improve the ability of light to couple out from the optical waveguide into hairs, particularly if the material of the cladding has a high refractive index.

In the embodiments shown in Figs. 4 and 5, a plurality of structures 16 (which in the embodiments discussed are cavities) are shown spaced regularly along the central optical axis X. As discussed below, more or fewer structures may be formed in the optical waveguide 4, depending on a number of factors, including the size of the structures, the size of the optical waveguide and the material from which the optical waveguide is formed. In Fig. 4, the structures extend substantially over the length of the portion of the optical waveguide 4 shown. However, in Fig. 5, the structures 16 extend over only part of the length of the optical waveguide 4. Specifically, the structures 16 may be formed in a portion of the optical waveguide 4 corresponding to the length of the cutting face 14, as shown in Fig. 5.

As noted above, the structures of the embodiments shown in the Figures comprise cavities. The structures 16 may, in other embodiments, comprise bubbles or voids. In embodiments in which the structures 16 comprise bubbles, each bubble may be filled with a gas, a liquid or a solid either at the time of formation of the bubbles or after their formation. In some embodiments, the bubbles may contain a gas which may be formed as a result of the formation of the bubbles. In embodiments in which the structures 16 comprise voids, each void may contain a substantial vacuum (i.e. no, or very little gas).

The shape of the structures 16 may be determined by the manner in which they are formed and, in some embodiments, the structures may be substantially spherical in shape. The surfaces of spherical structures act as negative lenses which serve to refract, reflect and/or scatter light, thereby increasing the angle of propagation of the light with respect to the optical axis. Structures of other shapes similarly refract, reflect and/or scatter light. Thus in other embodiments, structures of other, non-spherical shapes may be formed with in the optical waveguide 4.

As noted above, the structures 16 shown in the embodiments of Figs. 4 and 5 are spaced apart evenly along the optical axis X of the optical waveguide 4. In other words, the structures 16 are formed at regular intervals along at least part of the length of the optical waveguide 4. In some embodiments, however, the structures may be spaced in an irregular fashion, such that the distances between the structures 16 are not the same along the length of the cutting element 4. The spacing between the structures 16 and the size of the structures (e.g. the diameter of the structures) may be selected based on various factors, such as the length of the cutting element, the length of the cutting face of the cutting element, a diameter of the cutting element, a material from which the cutting element is formed, and a wavelength of light to be propagated through the optical waveguide. In general, the size (e.g. diameter) of each structure is smaller than the diameter of the optical waveguide and, preferably, smaller than the core of the optical waveguide. In other words, the structures 16 do not extend to the surface of the optical waveguide 4.

The arrangement of the structures 16 within the optical waveguide 4 may be selected to achieve a desired amount of refraction, reflection and/or scattering of the light propagating through the waveguide. If the structures cause the propagation direction of the light to be altered by too great an angle, then the redirected light may propagate at an angle which is too great to be retained within the core 18 by the effects of total internal reflection and, as such, the light may escape from the optical waveguide 4. Such light may not couple into hairs coming into contact with the cutting element 4, or may couple only into hairs coming into contact with a particular portion of the cutting element. Thus, the size of the structures and the spacing between adjacent structures may be selected to achieve the desired amount of refraction, reflection and/or scattering (i.e. that results in the most effective cutting ability of the cutting element). The arrangement of the structures 16 within the optical waveguide 4 is also important so that an even and consistent amount light is available to couple out of the cutting element over the entire length of the active portion of the cutting element (i.e. over the length of the cutting face 14). In some embodiments, the size of the structures is chosen to be relatively smaller than the size of the lowest-order mode of the optical waveguide 4. In this context, the size of the lowest-order mode is taken to be the FWHM, or the 1/e² value, of the Gaussian intensity profile of the lowest-order mode when viewed along the cross-section of the optical waveguide. In this way, the propagation modes of the light can be converted gradually from lower-order modes to higher-order modes, thereby causing a substantially constant intensity of light over the length of the cutting face 14 (i.e. the active cutting area of the cutting element 4).

In some embodiments, the arrangement of structures 16 within the optical waveguide 4 may be selected so as to cause a greater amount of light deflection at some positions in the cutting element compared other positions in the cutting element. For example, it may be desirable to create regions along the cutting face 14 (for example at the ends of the cutting face) where a relatively larger proportion of light is deflected towards the cutting face to be available to couple into hairs. In such scenarios, spacing between adjacent structures 16 may be smaller in regions corresponding to end portions of the cutting face 14 than in other regions in the cutting element 4.

In addition to a cutting element 4 having structures formed therein, the invention also provides a hair cutting device 2 for cutting hair on a body of a subject. The hair cutting device 2 comprises a light source 6 for generating laser light at one or more specific wavelengths corresponding to wavelengths absorbed by one or more chromophores in hair, and a cutting element 4 as described herein. The cutting element 4 is coupled to the light source 6. Such a hair cutting device 2 is discussed above with reference to Fig. 2.

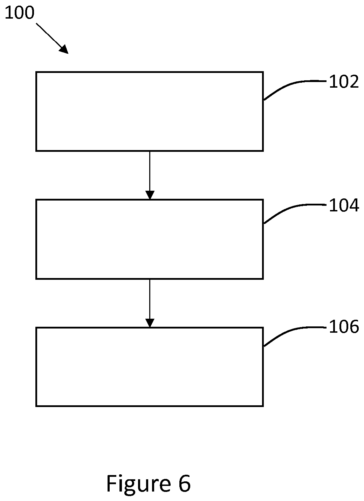

The invention also provides a method of manufacturing an optical waveguide cutting element 4 for use in a hair cutting device 2. Fig. 6 is a flowchart showing an example of such a method 100. The method 100 comprises providing (step 102) an optical waveguide 4 having an optical axis X; determining (step 104) a pattern in which to form a plurality of structures 16 within the optical waveguide; and focusing (step 106) a laser beam within the optical waveguide so as to form the plurality of structures along the optical axis in accordance with the determined pattern. The pattern may comprise an intended arrangement of the structures 16 within the optical waveguide, including, for example, details of the intended size of the structures (e.g. diameter, height, width, length and/or volume), and details of the intended spacing between adjacent structures. The pattern may, in some embodiments, be determined as a result of computational simulations and/or experimentation. In some embodiments, said determining may be based at least in part on at least one of: a length of the cutting element, a diameter of the cutting element, a material from which the cutting element is formed, and a wavelength of light to be propagated through the optical waveguide.

In some embodiments, the pattern may be stored in a storage medium/memory (not shown), and used to programme a computing device for controlling a laser source. The computing device may then control the laser source so as to direct and focus a laser beam into the optical waveguide 4 so as to create the structures 16 in accordance with the determined pattern. The laser source used to create the structures 16 may, in some embodiments, be a nanosecond or a femtosecond laser source. The focused laser beam may create a breakdown event in the optical waveguide 4, causing a structure to be formed.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. A cutting element for use in a hair cutting device, the cutting element comprising:
an optical waveguide having an optical axis and a sidewall, wherein a portion of the sidewall forms a cutting face for contacting hair;
wherein the optical waveguide includes a plurality of structures formed within the optical waveguide along the optical axis, the structures having a refractive index which is different to a refractive index of the optical waveguide.

2. A cutting element according to claim 1, wherein each structure comprises one of a cavity, a void or a bubble.

3. A cutting element according to claim 1 or claim 2, wherein each structure is substantially spherical.

4. A cutting element according to any of the preceding claims, wherein the refractive index of the structures is lower than the refractive index of the optical waveguide.

5. A cutting element according to any of the preceding claims, wherein the structures are regularly spaced along the optical axis of the optical waveguide.

6. A cutting element according to any of the preceding claims, wherein the structures are substantially the same size as one another.

7. A cutting element according to any of the preceding claims, wherein the optical waveguide comprises an optical fibre.

8. A cutting element according to any of the preceding claims, wherein the optical waveguide is formed from a material selected from a group comprising: quartz, sapphire, yttrium aluminium garnet, YAG, yttrium aluminosilicates, aluminosilicates, alkaline earth silicates, alkali aluminosilicates, rare-earth doped aluminosilicates, and oxyfluorides.

9. A cutting element according to any of the preceding claims, wherein each structure has a size which is smaller than a size of a lowest-order light propagation mode of the optical waveguide.

10. A hair cutting device for cutting hair on a body of a subject, the hair cutting device comprising:
a light source for generating laser light at one or more specific wavelengths corresponding to wavelengths absorbed by one or more chromophores in hair; and
a cutting element according to any of the preceding claims, the cutting element being coupled to the light source.

## Patentansprüche

1. Schneideelement zur Verwendung in einer Haarschneidevorrichtung, wobei das Schneideelement umfasst:
einen optischen Wellenleiter mit einer optischen Achse und einer Seitenwand, wobei ein Abschnitt der Seitenwand eine Schneidfläche zum Kontaktieren von Haaren bildet;
wobei der optische Wellenleiter eine Vielzahl an Strukturen enthält, die innerhalb des optischen Wellenleiters entlang der optischen Achse ausgebildet sind, wobei die Strukturen einen Brechungsindex aufweisen, der sich von einem Brechungsindex des optischen Wellenleiters unterscheidet.

2. Schneideelement nach Anspruch 1, wobei jede Struktur eine/n aus einem Hohlraum, einer Lücke oder einer Blase umfasst.

3. Schneideelement nach Anspruch 1 oder Anspruch 2, wobei jede Struktur im Wesentlichen kugelförmig ist.

4. Schneideelement nach einem der vorhergehenden Ansprüche, wobei der Brechungsindex der Strukturen niedriger als der Brechungsindex des optischen Wellenleiters ist.

5. Schneideelement nach einem der vorhergehenden Ansprüche, wobei die Strukturen regelmäßig entlang der optischen Achse des optischen Wellenleiters beabstandet sind.

6. Schneideelement nach einem der vorhergehenden Ansprüche, wobei die Strukturen im Wesentlichen die gleiche Größe zueinander aufweisen.

7. Schneideelement nach einem der vorhergehenden Ansprüche, wobei der optische Wellenleiter eine optische Faser umfasst.

8. Schneideelement nach einem der vorhergehenden Ansprüche, wobei der optische Wellenleiter aus einem Material ausgebildet ist, das aus einer Gruppe umfassend: Quarz, Saphir, Yttriumaluminiumgranat, YAG, Yttriumaluminosilicate, Aluminosilicate, Erdalkalisilicate, Alkalialuminosilicate, selten- erddotierte Aluminosilikate und Oxyfluoride ausgewählt ist.

9. Schneideelement nach einem der vorhergehenden Ansprüche, wobei jede Struktur eine Größe aufweist, die kleiner als eine Größe eines Lichtausbreitungsmodus niedrigster Ordnung des optischen Wellenleiters ist.

10. Haarschneidevorrichtung zum Schneiden von Haaren auf einem Körper eines Subjekts, wobei die Haarschneidevorrichtung umfasst:
eine Lichtquelle zum Erzeugen von Laserlicht bei einer oder mehreren spezifischen Wellenlängen, die Wellenlängen entsprechen, die von einem oder mehreren Chromophoren im Haar absorbiert werden; und
ein Schneideelement nach einem der vorhergehenden Ansprüche, wobei das Schneideelement mit der Lichtquelle gekoppelt ist.

## Revendications

1. Élément de coupe destiné à être utilisé dans un dispositif de coupe de cheveux, l'élément de coupe comprenant :
un guide d'ondes optiques comportant un axe optique et une paroi latérale, dans lequel une partie de la paroi latérale forme une face de coupe pour le contact avec les cheveux ;
dans lequel le guide d'ondes optiques inclut une pluralité de structures formées à l'intérieur du guide d'ondes optiques le long de l'axe optique, les structures comportant un indice de réfraction étant différent d'un indice de réfraction du guide d'ondes optiques.

2. Élément de coupe selon la revendication 1, dans lequel chaque structure comprend l'un des éléments suivants : une cavité, un vide ou une bulle.

3. Élément de coupe selon la revendication 1 ou la revendication 2, dans lequel chaque structure est substantiellement sphérique.

4. Élément de coupe selon l'une quelconque des revendications précédentes, dans lequel l'indice de réfraction des structures est inférieur à l'indice de réfraction du guide d'ondes optiques.

5. Élément de coupe selon l'une quelconque des revendications précédentes, dans lequel les structures sont régulièrement espacées le long de l'axe optique du guide d'ondes optiques.

6. Élément de coupe selon l'une quelconque des revendications précédentes, dans lequel les structures sont substantiellement de la même taille les unes aux autres.

7. Élément de coupe selon l'une quelconque des revendications précédentes, dans lequel le guide d'ondes optiques comprend une fibre optique.

8. Élément de coupe selon l'une quelconque des revendications précédentes, dans lequel le guide d'ondes optiques est formé d'un matériau choisi dans un groupe comprenant : le quartz, le saphir, le grenat d'yttrium-aluminium (YAG), les aluminosilicates d'yttrium, les aluminosilicates, les silicates alcalino-terreux, les aluminosilicates alcalins, les aluminosilicates dopés aux terres rares et les oxyfluorures.

9. Élément de coupe selon l'une quelconque des revendications précédentes, dans lequel chaque structure a une taille qui est inférieure à une taille d'un mode de propagation de la lumière d'ordre le plus bas du guide d'ondes optiques.

10. Dispositif de coupe de cheveux pour la coupe de cheveux sur un corps d'un sujet, le dispositif de coupe de cheveux comprenant :
une source de lumière pour générer une lumière laser à une ou plusieurs longueurs d'onde spécifiques correspondant aux longueurs d'onde absorbées par un ou plusieurs chromophores dans les cheveux ; et
un élément de coupe selon l'une quelconque des revendications précédentes, l'élément de coupe étant couplé à la source de lumière.
